# EUROPEAN PATENT SPECIFICATION

(11) **EP 3 779 396 B1**
(45) Date of publication and mention of the grant of the patent: **23.08.2023**
(21) Application number: 19020587.2
(22) Date of filing: 18.10.2019
(51) Int. Cl.: G01N 1/04, A01B 15/18, A01B 79/00, G01N 1/08

(54) **MULTIFUNCTIONAL UNIT AND METHOD FOR READING AND PROCESSING SOIL PARAMETERS**
MULTIFUNKTIONALE EINHEIT UND VERFAHREN ZUM LESEN UND VERARBEITEN VON BODENPARAMETERN
UNITÉ MULTIFONCTIONNELLE ET PROCÉDÉ DE LECTURE ET DE TRAITEMENT DE PARAMÈTRES DE SOLS

(30) Priority: 16.08.2019 RO 201900501
(43) Date of publication of application: 17.02.2021
(73) Proprietor: Institutul National de Cercetare Dezvoltare pentru Masini si Instalatii destinate Agriculturii si Industriei Alimentare-INMA, Bucuresti (RO)
(72) Inventor: Muraru, Vergil Marian, Bucuresti (RO); Muraru, Sebastian Lucian, Bucuresti (RO); Constantin, Nicolae, Bucuresti (RO); Muraru, Ionel Cornelia, Bucuresti (RO); Ganea, Christu Ioan, Bucuresti (RO)
(74) Representative: Strenc, Alexandru Cristian

(56) References cited:
- US-A- 6 138 590
- US-B1- 6 608 672
- US-B2- 7 216 555

## Description

### Technical Field

This invention is referring to a multifunctional unit, which is mounted on a frame of a trailed agricultural equipment, intended for soil sampling, reading and computer processing of soil parameters, for example pH and humidity, as well as GPS coordinates.

### Background Art

In practice different measuring instruments, devices or equipment are used, such as the one used by Veris Technology from the USA presented in the U.S. Patent No. 7216555B2 "System and method for mobile soil sampling", which is used for measuring the soil pH. This equipment is operated by means of a hydraulic system, the soil sample being taken up and raised to the pH sensor level. The sampling system moves vertically relative to the mobile equipment and the sensors are fixed relative to the same equipment. The solution has some limitations due to the lack of flexibility in using several types of sensor, the used hydraulic power source requiring certain high energy consumption and the long measurement time.

The solution in the patent document US 6 138 590 A, consists of a real time complex in situ soil resistivity sensor and a prescription agricultural chemical delivery system, which includes a plurality of ground- engaging tools in association with individual soil electrode arrays which measure complex solute and matrix resistivity levels. The solution does not include pH determinations, but includes a sensor for measuring the temperature of the soil. The proposed equipment is complex, and oriented towards including complex soil resistivity, not always suitable for common agricultural activities.

The solution in the patent document US 6 608 672 B1 consists in an optical soil survey device to survey the optical characteristics of a given underground soil, a soil survey device to survey the soil, a soil survey system and a mobile soil survey vehicle to survey the soil. The solution does not include pH determinations for soil but however contains an infrared-emitting thermometer (infrared thermocouple) which surveys the temperature of the survey surface under observation. The data processing unit is rather complex and sophisticated, being dedicated to analyse and process a mixture of images and physical parameters.

There are also manual soil sampling kits that are stored in containers and transported to the laboratory for analysis. Soil temperature determination can't be done with these kits, but there are tools that manually determine the soil temperature in situ as for example the SM150T from Delta-T Devices Ltd in the UK. This device must be connected to a data logger from Delta-T Devices Ltd such as Data Logger DL2e for reading and storing of measured data.

Disadvantages of these solutions are:
- hydraulic drive system requires a hydraulic power source;
- possibility of measurement is limited to a single parameter - pH;
- requires the interruption of work for additional operations before and after measurements, such as soil moisture for parameter determination, and subsequent cleaning of the working body;
- measurement time is relatively high due to the vertical travel of sample taker.

### Summary of invention

Technical problem solved by the proposed solution is the identification in a continuous flow of a large volume of data constituting the characteristic parameters of the soil through a multifunctional section, before beginning the agricultural works for the purpose of optimization of parameters which will lead to increased efficiency and harvests.

Multifunctional unit for reading and processing of soil parameters according to the invention is made up of a "V" form coulter mounted on a frame support of a section, which is provided at the bottom with a tube with a spheric head having at the previous side a hole for taking soil samples, a shutter for stopping the soil inside the coulter for a transducer with sensors to read the parameters from the soil sample, the shutter and the transducer being solidary and actuated by a linear actuator.

After reading the shutter rises and allows the movement of another soil sample through a gutter, the data being transmitted to an electronic system for the processing and storage of GPS data, portable computer or similar.

By applying the invention, the following advantages are obtained:
- increased accuracy of the data recorded in real time, together with the location of their geographical location (GPS);
- the possibility to register several categories of data in a single pass;
- the working process is approximately continuous, without technological interruptions;
- reduced manufacturing costs by using a simple electrical control scheme with actuator;
- reduced measurement time allows work at higher speeds or the increase of the density of the measuring points on the surface unit.

### Brief description of drawings

The following is an example of the realization of the invention related to 1, 2 and 3 figures which represent:

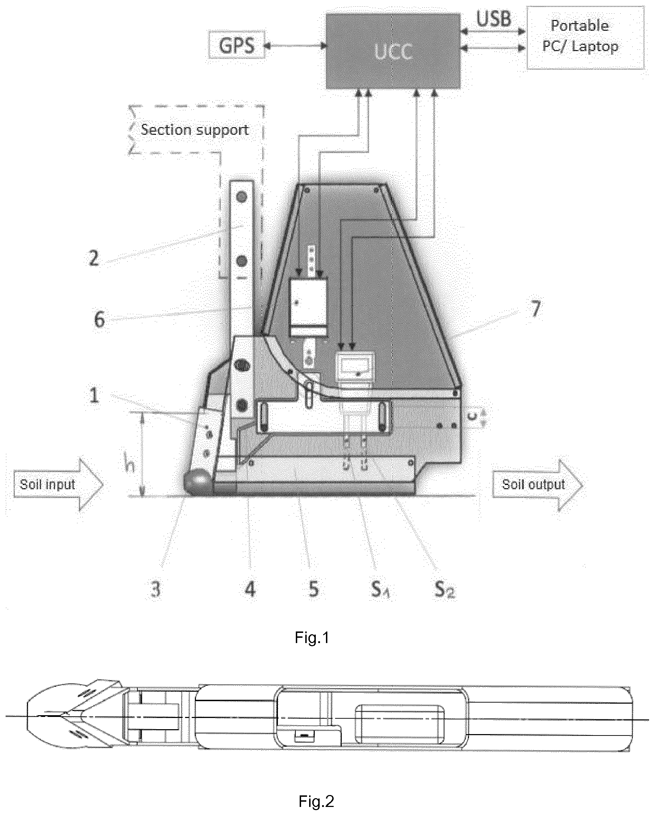

- Fig. 1 - Multifunctional unit for reading and processing soil parameters - general assembly scheme;
- Fig. 2 - Multifunctional unit for reading and processing soil parameters - top view;

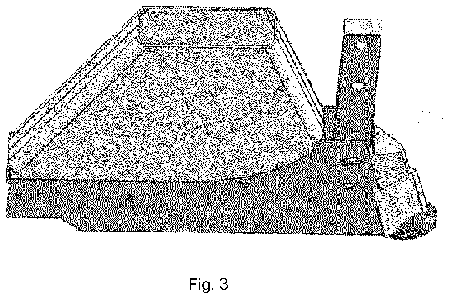

- Fig. 3 - Multifunctional unit for reading and processing soil parameters - 3D representation.

### Description of embodiments

Multifunctional section for reading soil parameters mounted on a frame of a trailed agricultural equipment is made up of coulter 1 in the form of a lying "V", with the tip towards the forward direction, of a mounting bracket 2 on the frame, the tube 3 with spheric head provided with a hole for taking soil samples, the shutter 4 which stops the soil inside the coulter for reading the parameters and allows to take the soil sample, gutter 5 through which the soil circulates or stands from which the samples are taken, linear actuator 6 which actuates the shutter 4 vertically between the ends of a slider on the c travel, the transducer 7 for determining the temperature and pH of the soil through sensors S1 and S2, mounted solidary with the shutter 4, as well as an electronic data - processing and storage system (command and control unit - UCC, GPS, portable calculator, etc.).

During the work, the multifunctional section is deposited in the soil at a h depth so that through the tube with spheric head 3 and gutter 5 the displaced soil penetrates and moves for sampling. Depending on the sampling distance of the soil samples, the UCC, based the information received from the GPS triggers the linear actuator 6 which controls the lowering of shutter 4 and the stop of the soil in the tube with spheric head 3. Simultaneously with the stop of the taken soil in the tube with spheric head 4, the soil in the gutter 5 stops, and the transducer 7, which is mounted solidary on the shutter 4, descends into the soil located in the gutter 5. The system is maintained in this position long enough for recording soil parameters (pH and humidity) through sensors S1 and S2 and the GPS coordinates, in UCC memory.After the end of the recording, the shutter 4 rises vertically in the initial position, together with transducer 7 and awaits a new sampling command for the next soil sample. At the end of the working period, the measured parameters are downloaded via the USB interface in the memory of a portable computer for further processing.

## Claims

1. Multifunctional unit for reading and processing of soil parameters of soil sample, mounted on a frame of a trailed agricultural equipment, which includes an electronic processing and a storage system of the command and control unit (UCC), a GPS and a portable computer, a coulter (1) in the form of lying "V", with the tip of the coulter oriented from a mounting bracket (2) on the frame towards a forward direction, the coulter comprising a tube (3) with a spheric head provided with a hole for taking soil sample, a gutter (5) through which the soil circulates or stands from which the samples can be taken, a shutter (4) configured to stop the soil inside the gutter for reading of the parameters and allows the soil sample to be taken into the gutter (5), a linear actuator (6) configured for actuating the shutter (4) vertically between the end positions of a slider, a transducer (7) for determining soil humidity and soil temperature and a soil pH through two sensors (S1, S2), which are mounted in solidarity with the shutter (4).

2. Method for reading and processing soil parameters implemented in the multifunctional unit according to the claim 1, **characterized in that** it involves the following the next steps:
- moving the multifunctional unit into the ground for sampling soil such that the dislocated soil enters and moves at a depth (h) so that through the tube with the spheric head (3) and the gutter (5) ;
- triggering by the UCC, based on the information received from GPS, and depending on the sampling distance of the soil samples, of the linear actuator (6) which controls the lowering of the shutter (4) and the stop of the soil in the tube with spheric head (3);
- simultaneously with the stop of the soil taken in the tube with spheric head (3), stopping the soil located in the gutter (5), and the lowering of the transducer (7) which is mounted solidary on the shutter (4) in the soil stopped in the gutter (5);
- keeping the system in this position long enough for registration in the memory of the UCC of the specific parameters of the soil, respectively the pH and the humidity, through sensors (S1, S2) and GPS coordinates;
- lifting, after completing the recording, of the shutter (4) vertically in its initial position, together with the transducer (7) and awaiting a new sampling command for taking the next soil sample;
- downloading the parameters measured in the memory of a portable computer for further processing.

## Patentansprüche

1. Multifunktionaler Einheit zum Lesen und Verarbeiten von Bodenparametern von Bodenproben, montiert auf einem Rahmen einer gezogenen landwirtschaftlichen Gerät, der umfasst ein elektronisches Datenverarbeitungs- und Speichersystem der Befehls- und Steuereinheit (UCC), ein GPS und ein tragbarer Computer, ein Schar (1) in Form eines liegenden "V", dessen Spitze von einer Montagehalterung (2) am Rahmen nach vorne gerichtet ist, wobei das Schar ein Rohr (3) mit kugelförmigem Kopf umfasst dass mit einem Loch zur Entnahme von Bodenproben versehen ist, eine Rinne (5), durch die der Boden zirkuliert, oder steht, aus der die Proben entnommen werden können, einem Verschluss (4), der so konfiguriert ist dass er der den Boden innerhalb der Rinne zum Ablesen der Parameter stoppt und ermöglicht einer in die Rinne (5) zu entnehmenden Bodenprobe, einem Linearantrieb (6), der der zum vertikalen Betätigen des Verschlusses (4) zwischen den Endpositionen eines Schiebers configuriert ist, einem Messwertaufnehmer (7) zur Bestimmung der Bodenfeuchtigkeit und der Bodentemperatur und einem Boden pH-Wert durch zwei Sensoren (S1, S2), die solidarisch mit dem Verschluss (4) montiert sind.

2. Verfahren zum Lesen und Verarbeiten von Bodenparametern, das im Multifunktionaleeinheit nach Anspruch 1 implementiert ist, **dadurch gekennzeichnet, dass** es die folgenden Schritte umfasst:
- Bewegen der Multifunktionseinheit in den Boden zur Probenahme von Boden, so dass der verlagerte Boden in eine Tiefe (h) eindringt und sich durch das Rohr mit dem kugelförmigen Kopf (3) und der Rinne (5) bewegt;;
- Auslösen des Linearantriebs (6) durch das UCC, basierend auf den vom GPS erhaltenen Informationen, der das Absenken des Verschlusses (4) und den Stopp des Bodens im Rohr mit Kugelkopf (3) abhängig von der Entnahmeentfernung der Bodenproben steuert;
- Stoppen, gleichzeitig mit dem Stoppen des in das Rohr mit kugelförmigem Kopf (3) aufgenommenen Bodens und des in der Rinne (5) befindlichen Bodens und dem Absenken des Messwertaufnehmer s (7), der fest am Verschluss (4) montiert ist; im Boden in der Rinne (5) angehalten;
- Halten des Systems in dieser Position lange genug, um im Speicher des UCC die spezifischen Parameter des Bodens, bzw. den pH-Wert und die Feuchtigkeit, über die Sensoren (S1,S2) und GPS-Koordinaten zu registrieren;
- Nach Abschluss der Aufzeichnung wird der Verschluss (4) zusammen mit dem Messwertaufnehmer (7) vertikal in die Ausgangsposition gehoben und auf einen neuen Probenahmebefehl zur Entnahme der nächsten Bodenprobe gewartet;
- Herunterladen der gemessenen Parameter in den Speicher eines tragbaren Computers zur weiteren Verarbeitung.

## Revendications

1. Unité multifonctionnelle de lecture et de traitement des paramètres de sols d'un échantillon de sol, montée sur un châssis d'un un matériel agricole traîné, qui comprend un système électronique de traitement et de stockage des données de l'unité de commande et de contrôle (UCC), un GPS et un ordinateur portable, un soc (1) en forme de "V" couché, avec la pointe du soc orientée à partir du support (2) fixé sur le châssis vers l'avant, le soc comprenant un tube (3) a tête sphérique munie d'un trou de prélèvement d'échantillon de sol , une gouttière (5) à travers laquelle le sol circule ou se dresse à partir de laquelle les échantillons peuvent être prélevés, un obturateur (4) configuré pour arrêter le sol à l'intérieur de la gouttière pour la lecture des paramètres et permet de prélever l'échantillon de sol dans la gouttière (5), un actionneur linéaire (6) configuré pour actionner l'obturateur (4) verticalement entre les extrémités d'un curseur, un transducteur (7) pour déterminer l'humidité et la température du sol et un pH du sol grâce à travers deux capteurs (S1,S2, qui sont montés solidaires de l'obturateur (4).

2. Méthode de lecture et de traitement des paramètres du sol mis en oeuvre dans l'unité multifonctionnelle selon la revendication 1, **caractérisé en ce qu'**il consiste à suivre les étapes suivantes :
- enfoncer l'unité multifonctionnelle dans le sol pour échantillonner le sol, de sorte que le sol disloqué pénètre et se déplace à une profondeur (h) 'à travers le tube avec la tête sphérique (3) et la gouttière (5) ;
- déclenchement par l'UCC, en fonction des informations reçues du GPS, de l'actionneur linéaire (6) qui commande la descente de l'obturateur (4) et l'arrêt du sol dans le tube à tête sphérique (3), en fonction de la distance de prélèvement des échantillons de sol ;
- arrêt, simultanément à l'arrêt du sol prélevé dans le tube à tête sphérique (3), du sol situé dans la gouttière (5), et à l'abaissement du transducteur (7) qui est monté solidaire sur l'obturateur (4) dans le sol arrêtée dans la gouttière (5) ;
- maintenir le système dans cette position suffisamment longtemps pour l'enregistrement dans la mémoire de l'UCC des paramètres spécifiques du sol, respectivement le pH et l'humidité, à travers les capteurs (S1,S2) et les coordonnées GPS ;
- remontée, après avoir terminé l'enregistrement, de l'obturateur (4) à la verticale en position initiale, en même temps que le transducteur (7) et en attente d'une nouvelle commande d'échantillonnage pour prélever l'échantillon de sol suivant ;
- téléchargement des paramètres mesurés dans la mémoire d'un ordinateur portable pour un traitement ultérieur.
